# EUROPEAN PATENT SPECIFICATION

(11) **EP 3 672 494 B1**
(45) Date of publication and mention of the grant of the patent: **29.11.2023**
(21) Application number: 18759280.3
(22) Date of filing: 17.08.2018
(51) Int. Cl.: A61B 8/08

(54) **DETECTION OF B-LINES IN LUNG ULTRASOUND**
ERKENNUNG VON B-LINIEN IM LUNGENULTRASCHALL
DÉTECTION DE LIGNES B DANS DES ULTRASONS PULMONAIRES

(30) Priority: 21.08.2017 WO PCT/CN2017/098272; 13.11.2017 EP 17201269
(43) Date of publication of application: 01.07.2020
(73) Proprietor: Koninklijke Philips N.V., 5656 AG Eindhoven (NL)
(72) Inventor: XU, Jingping, 5656 AE Eindhoven (NL); RAJU, Balasundar, Iyyavu, 5656 AE Eindhoven (NL); WANG, Shougang, 5656 AE Eindhoven (NL)
(74) Representative: Philips Intellectual Property & Standards
(86) International application number: PCT/EP2018/072370
(87) International publication number: WO 2019/038210

(56) References cited:
- US-A1- 2017 086 790
- MOSHAVEGH RAMIN ET AL: "Novel automatic detection of pleura and B-lines (comet-tail artifacts) on in vivo lung ultrasound scans", PROGRESS IN BIOMEDICAL OPTICS AND IMAGING, SPIE - INTERNATIONAL SOCIETY FOR OPTICAL ENGINEERING, BELLINGHAM, WA, US, vol. 9790, 1 April 2016 (2016-04-01), pages 97900K-97900K, XP060065636, ISSN: 1605-7422, DOI: 10.1117/12.2216499 ISBN: 978-1-5106-0027-0
- LAURA J. BRATTAIN ET AL: "Automated B-Line Scoring on Thoracic Sonography", JOURNAL OF ULTRASOUND IN MEDICINE., vol. 32, no. 12, 1 December 2013 (2013-12-01), pages 2185-2190, XP055437464, United States ISSN: 0278-4297, DOI: 10.7863/ultra.32.12.2185

## Description

### TECHNICAL FIELD

This application pertains to ultrasound imaging and more specifically to systems and methods for detecting B-lines in images of the lungs, evaluating B-lines across multiple image frames, and displaying B-line information.

### BACKGROUND

Lung ultrasound can be performed by positioning the ultrasound transducer both longitudinally, perpendicular to the ribs, and obliquely, along the intercostal spaces. Among the various features evaluated via lung ultrasound to diagnose conditions such as pneumothorax ("PTX"), pneumonia, pulmonary edema and others, are visual artifacts known as B-lines. B-lines are discrete/fused vertical hyperechoic reverberations that typically extend downward, e.g., closer to maximum imaging depth, from the pleural line, which marks the interface between the chest wall and the lung. B-line scoring may be critical to characterizing lung diseases, diagnosing PTX, and also estimating extravascular lung water, which may be indicative of several lung conditions. As such, inaccurate B-line assessment may lead to inaccurate diagnoses and evaluation of lung conditions. Identifying B-Lines may be difficult and time-consuming for dynamic frame-to-frame live imaging, especially for inexperienced users manipulating an ultrasound probe while subjectively interpreting and manually counting B-lines within each ultrasound image from lung ultrasound image sequences. Techniques for a more precise, user-friendly approach to detect and/or display B-lines may be desired in a wide range of medical settings, e.g., clinical applications at emergency treatment centers, intensive care units ("ICU"), and critical care units, to name a few. One example of an ultrasound device that detects B-lines is given in US 2017/0086790 A1.

### SUMMARY

The invention is defined by the independent claims. Dependent claims define advantageous embodiments.

Provided herein are ultrasound systems and methods for improved B-line detection and assessment in the lungs. Various examples involve systems configured to receive and evaluate ultrasound echoes embodied in a plurality of image frames to identify and display a target image frame containing the most and/or brightest B-lines. In some examples, the target frame could be used as a frame of reference, e.g., a representation, in final reporting to end-users, indicating when and where (left or right side, upper or lower) the maximum B-line event happens. This information may add value in clinical reports for quick decision-making in emergency situations. Systems may include an ultrasound probe configured to receive ultrasound echoes, a signal processor configured to generate a plurality of image frames, and a data processor configured to identify a pleural line and region of interest proximate, e.g., below, each pleural line. Within each region of interest, the data processor may be configured to identify one or more candidate B-lines. Candidates may be excluded or selected as legitimate B-lines by evaluating one or more parameters, which may include a level of intensity uniformity, a coherent line length, a starting location, and/or an ending location of each candidate B-line. Systems may also include a user interface configured to display target image frames and various B-line characteristics determined by the data processor operating in tandem with the ultrasound probe. The systems disclosed herein may be automated and performed in real time, thereby reducing examination time and interpretive error.

### BRIEF DESCRIPTION OF THE DRAWINGS

Fig. 1 is a block diagram of an ultrasound imaging system in accordance with the principles of the present disclosure;
Fig. 2A is a lung ultrasound image taken with an ultrasound probe in accordance with the principles of the present disclosure;
FIG. 2B is the lung ultrasound image of FIG. 2A including an indication of a pleural line and a B-line;
Fig. 2C is the lung ultrasound image of FIG. 2B including an indication of additional B-lines;
Fig. 2D is a graph indicating a width and amplitude of the B-lines indicated in Fig. 2C;
Fig. 3A is an intensity map of B-lines detected over time in a plurality of image frames;
Fig. 3B is a line trace map of the intensity map shown in FIG. 3A;
Fig. 4 is a graphical representation of a B-line scoring system;
Fig. 5A is a diagram of multiple sub-regions within a region of interest of a subj ect;
Fig. 5B is a table reporting qualitative B-line scores for regions of interest shown in Fig. 5A;
Fig. 5C is a table reporting a quantitative B-line score for each region of interest shown in Fig. 5A;
Fig. 6 is a block diagram of an ultrasound imaging system in accordance with the principles of the present disclosure;
Fig. 7 is a lung ultrasound image taken at a target image frame in accordance with the principles of the present disclosure; and
Fig. 8 is a block diagram of an ultrasound imaging method in accordance with principles of the present disclosure.

### DETAILED DESCRIPTION

The following description of certain exemplary embodiments is merely exemplary in nature and is in no way intended to limit the invention or its applications or uses. In the following detailed description of embodiments of the present systems and methods, reference is made to the accompanying drawings which form a part hereof, and in which are shown by way of illustration specific embodiments in which the described systems and methods may be practiced. These embodiments are described in sufficient detail to enable those skilled in the art to practice the presently disclosed systems and methods, and it is to be understood that other embodiments may be utilized and that structural and logical changes may be made without departing from the scope of the present system. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

The present technology is also described below with reference to block diagrams and/or flowchart illustrations of methods, apparatus (systems) and/or computer program products according to the present embodiments. It is understood that blocks of the block diagrams and/or flowchart illustrations, and combinations of blocks in the block diagrams and/or flowchart illustrations, may be implemented by computer executable instructions. These computer executable instructions may be provided to a processor, controller or controlling unit of a general purpose computer, special purpose computer, and/or other programmable data processing apparatus to produce a machine, such that the instructions, which execute via the processor of the computer and/or other programmable data processing apparatus, create means for implementing the functions/acts specified in the block diagrams and/or flowchart block or blocks.

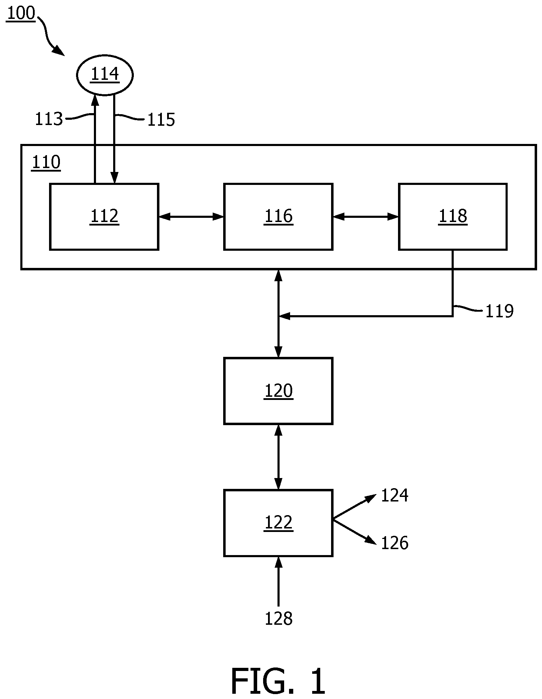

Fig. 1 shows an ultrasound system 100 configured to identify and display a target image frame for visualizing and evaluating B-lines in accordance with the present disclosure. As shown, the system 100 includes an ultrasound data acquisition unit 110. The ultrasound data acquisition unit 110 includes an ultrasound probe which includes an ultrasound sensor array 112 configured to transmit ultrasound signals or beams 113 into a region 114 of a subject, e.g., the lungs, and receive ultrasound signals or echoes 115 responsive to the transmitted beams. As further shown, the ultrasound data acquisition unit 110 includes a beamformer 116 and a signal processor 118, which are configured to generate a plurality of discrete image frames 119 from the ultrasound echoes 115 received at the array 112. The system 100 also includes a data processor 120, e.g., a computational module or circuity, configured to detect and evaluate candidate B-lines based on the ultrasound echoes 115 received at the array 112 and processed by the signal processor 118. In some embodiments, the system 100 includes at least one user interface 122 coupled with the data processor 120. The user interface 122 may display various images 124 of the region being scanned, e.g., target image frames containing B-lines, live ultrasound images obtained while the scan is performed, and/or pictorial representations of B-line distributions across a region of the subject being scanned. The user interface 122 may also be configured to display one or more indicators 126, which may embody one or more types of information regarding the existence and/or characteristics of identified B-lines. The user interface 122 may also be configured to receive a user input 128 at any time before, during, or after an ultrasound scan. The configuration of the system 100 shown in FIG. 1 may vary. For instance, the system 100 can be stationary or portable. Various portable devices, e.g., laptops, tablets, smart phones, or the like, may be used to implement one or more functions of the system 100. In examples that incorporate such devices, the ultrasound sensor array 112 may be connectable via a USB interface, for example.

The ultrasound data acquisition unit 110 may be configured to acquire ultrasound data for one or more regions of interest selectable by a user, e.g., a sonographer, clinician or ultrasound technician. According to embodiments of the present disclosure, the region of interest is a chest region encompassing one or both lungs. The ultrasound sensor array 112 may include at least one transducer array configured to transmit and receive ultrasonic energy. A variety of transducer arrays may be used, e.g., linear arrays, convex arrays, or phased arrays. The number and arrangement of transducer elements included in the sensor array 112 may vary in different examples. For instance, the ultrasound sensor array 112 may include a 1D or 2D array of transducer elements, corresponding to linear array and matrix array probes, respectively. The 2D matrix arrays may be configured to scan electronically in both the elevational and azimuth dimensions (via phased array beamforming) for 2D or 3D imaging. In some examples, a 2D matrix array may be configured to perform sub array beamforming using a microbeamformer, for example as described in U.S. Pat. No.: 6,013,032 (Savord). One-dimensional arrays may be configured to scan 2D images electronically (via phased array beamforming) or additionally be mechanically swept across a region of interest in an orthogonal direction to the electrically scanned dimension in order to create 3D images.

The data acquisition unit 110 may also include a beamformer 116, e.g., comprising a microbeamformer or a combination of a microbeamformer and a main beamformer, coupled to the ultrasound sensor array 112. The beamformer 116 may control the transmission of ultrasonic energy, for example by forming ultrasonic pulses into focused beams. The beamformer 116 may also be configured to control the reception of ultrasound signals such that discernable image data may be produced and processed with the aid of other system components. The role of the beamformer 116 may vary in different ultrasound probe varieties. In some embodiments, the beamformer 116 may comprise two separate beamformers: a transmit beamformer configured to receive and process pulsed sequences of ultrasonic energy for transmission into a subject, and a separate receive beamformer configured to amplify, delay, and/or sum received ultrasound echo signals. In some embodiments, the beamformer 116 may comprise a microbeamformer operating on groups of sensor elements for both transmit and receive beamforming, coupled to a main beamformer which operates on the group inputs and outputs for both transmit and receive beamforming, respectively.

As further shown in FIG. 1, at least one processor, such as signal processor 118, may be communicatively, operatively, and/or physically coupled with the sensor array 112. The signal processor 118 included in FIG. 1 is shown as an internal component of the data acquisition unit 110. In some embodiments, the signal processor 118 may comprise a separate component. The signal processor 118 may be configured to receive ultrasound data embodying the ultrasound echoes 115 received at the sensor array 112. From this data, the signal processor 118 generates a plurality of image frames 119 as a user scans the region 114 of the subject. In operation, the probe containing the ultrasound sensor array 112 may be moved over the surface of the region 114 to collect image data at multiple locations. The user may pause at one or more locations, keeping the sensor array 112 stationary while a series of image frames may be generated based on the ultrasound echoes 115 received at the acquisition unit 110. In this manner, image frames 119 spanning at least one respiratory cycle (preferably two or more cycles if time permits), may be collected at each location examined by the user, which may collectively span the entire chest region, including both lungs. The number of discrete locations may vary depending on the objectives of the user and the clinical setting. For instance, in the ER/ICU setting, about 4 to about 6 locations may be examined, while internal medicine applications may involve a more thorough examination of about 25 to about 35 locations. In various embodiments, the number of locations may range from about 1 to about 40, about 26 to about 34, about 25 to about 30, about 2 to about 30, about 4 to about 20, or about 6 to about 8 locations. By collecting multiple image frames 119 at each location, the system 100 may detect movement and/or changes in the shape of one or more B-lines, each of which may often occur during respiration, as the lungs expand and contract. For instance, discrete B-lines in one frame may fuse during respiration to appear as a single, wider B-line in a subsequent frame.

The number of image frames 119 generated by the signal processor 118 at a given location may vary depending on the ultrasonic pulse rate of the sensor array 112 and the length of time spent at each location. In some examples, the frame (pulse) rate may range from about 20 Hz to about 100 Hz, about 25 Hz to about 80 Hz, about 30 Hz to about 60 Hz, about 35 Hz to about 50 Hz, about 40 to about 48 Hz, or about 42 Hz to about 46 Hz. Higher pulse rates may enable more detailed data collection, such that the number and/or intensity of B-lines contained within the eventually-identified target image frame may be maximized. Accordingly, the pulse rate may be increased for more thorough inspection, which may be necessary to detect small changes in B-line number and/or conformation, and/or transient B-line features that change rapidly during respiration. The length of time spent at each location may also vary, ranging from about 2 to about 6 seconds depending on the respiratory rate of the subject being scanned. The total number of image frames generated at a discrete location may range from about 40 to about 600.

Using the image frames 119 generated by the signal processor 118, the data processor 120 may be configured to perform several operations to identify B-lines and/or select a target image frame for further examination and/or display. For instance, because B-lines begin at the pleural line, the data processor 120 may identify a pleural line in each of the plurality of image frames (if a pleural line is present in each frame). Identifying the pleural line may be necessary, in some examples, to reliably distinguish the B-lines from other hyperechoic features that, while vertically oriented, may not begin at the pleural line. Various pleural line identification techniques may be implemented by the data processor 120 to perform this operation. For instance, the data processor 120, in conjunction with the other components of the system 100, may perform any of the automated processing techniques disclosed in related US Patent Application US 2020/0060642 A1 titled "Target Probe Placement for Lung Ultrasound" and naming Balasundar et al.. In some examples, the data processor 120 may additionally evaluate the intensity and/or clarity of the pleural line detected in each image frame, and may compare multiple image frames to prioritize stronger pleural lines for further processing. In some embodiments, the data processor 120 may implement a Hough transform to identify one or more pleural lines. The data processor 120 may apply various intensity thresholding techniques to identify pleural lines and the boundaries thereof. Embodiments may also include one or more techniques for pleural line identification described in another related US Patent Application US 11191518 B2 titled "Ultrasound System and Method for Detecting a Lung Sliding" and naming Wang, Shougang; Raju, Balasundar; Xu, JingPing; Zhou, Shiwei; Gades, Tony; and Poland, McKee.

The data processor 120 may be further configured to define a region of interest proximate one or more of the identified pleural lines. Narrowing the area within each image frame to a defined region of interest may minimize unnecessary processing and/or reduce the number of false positives, e.g., B-lines detected by the system 100 that are not actually B-lines. The region of interest may include the region below the pleural line, extending downward away from the ribs. The size of the region of interest may vary, and may be based at least in part on an average B-line length determined through a sample of B-line images and/or published clinical data. Because B-lines may be defined as the vertical lines that begin at the pleural line and extend to the bottom of the region of interest, regions of interest spanning an insufficient depth may be overly inclusive. Relative to the surface of the subject being imaged, the region of interest may extend to a depth of about 2 cm to about 8 cm, about 3 cm to about 6 cm, about 3 cm to about 5 cm, or about 2.5 cm to about 3.5 cm.

Within the region of interest, the data processor 120 is configured to identify one or more candidate B-lines in each image frame. Candidates may include vertical, hyperechoic lines beginning at the pleural line. Out of the pool of candidate B-lines, the data processor 120 identifies legitimate B-line(s) by evaluating one or more parameters of each candidate, which involve applying one or more B-line classification rules programmed into the data processor. Parameters include any movement of each candidate detected across multiple image frames and may include the starting point of each candidate, the intensity of each candidate and/or the end location or length of each candidate. These parameters are based on several B-line characteristics. For example, as mentioned, B-lines begin at the pleural line. Accordingly, the data processor 120 may exclude any vertical lines that do not begin at the pleural line. In addition, each B-line may extend to the bottom of the region of interest and may have a relatively uniform intensity along the length of the line, such that the line is coherent along its length and does not fade in some examples. B-lines may also have an approximately uniform width along the length of each line. The data processor 120 may be configured to measure each of these parameters within the image frames 119 by applying, in some examples, thresholding techniques to determine the borders of each B-line. The lateral width of each B-line candidate may be measured at various points along the length of each line, and the measured widths compared to determine whether candidate B-lines maintain an approximately equal width. The data processor 120 may also be configured to measure the intensity of each B-line via B-mode image processing. Comparing the intensity levels at multiple locations along the length of each individual B-line candidate may enable the data processor to evaluate the uniformity of B-line intensity. Candidates having a level of uniformity above a specified threshold may be selected as B-lines. Further, B-lines often move as a subject breathes. As a result, B-lines may appear in different positions between any two sequentially-collected frames. Candidate B-lines are thus be compared across multiple image frames to detect movement from frame to frame.

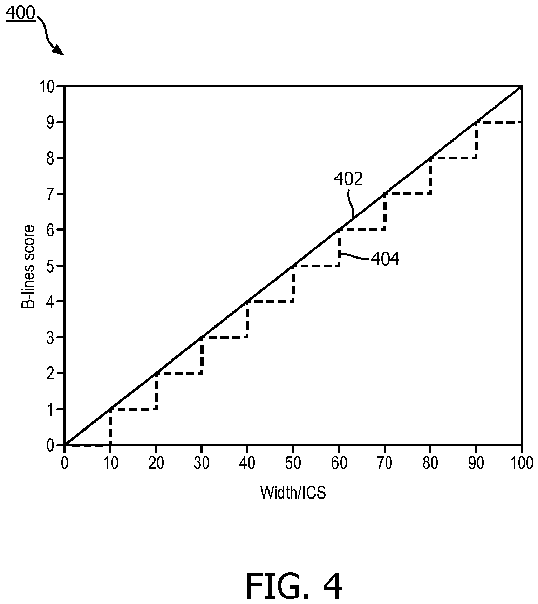

The data processor 120 may perform additional operations to determine the characteristics of one or more B-lines identified within each image frame. For example, the data processor 120 may determine the number of B-lines, the width of each B-line, the distance between one or more pairs of B-lines, and/or the width of the intercostal space (lateral distance between an adjacent pair of ribs) in each frame. The data processor 120 further determines a B-line score. The B-line score may be calculated in various ways. For instance, some embodiments may involve calculating a B-line score by combining two or more measured parameters, e.g., width, intensity, uniformity, number, density, etc., and forming a composite value. The B-line score is based at least in part on the level of B-line coverage within at least one intercostal space. According to such examples, a higher B-line score may reflect a greater amount of B-line coverage, e.g., B-line coverage of 80% produces a higher B-line score than a B-line coverage of only 20% (see Fig. 4 for additional explanation).

The data processor further selects a target image frame which may be used for closer examination and/or display by identifying the image frame that maximizes a number and/or an intensity of B-lines relative to the other image frames generated. Of all the image frames collected at a specific location, the target image frame may be the frame with the strongest B-line presence, and may thus be the optimal image frame (compared to the other frames). In particular, the present disclosure involves selecting a target image frame by identifying the image frame having the highest B-line score. In some embodiments, multiple target image frames may be selected, each target image frame corresponding to a discrete location on a subject's chest at which a series of image frames were collected. Each target image frame may thus correspond to a discrete sub-region that may be displayed on the user interface 122. The data processor 120 may be further configured to automatically save a copy of each target image frame to a memory storage device for later viewing. Archiving target image frames may also facilitate streamlined billing practices.

As further shown in Fig. 1, the system 100 may include at least one user interface 122. The user interface may be operatively, physically, and/or communicatively coupled with the ultrasound data acquisition unit 110 and the data processor 120. The user interface 122 may be configured to receive manual, electronic, and/or wireless input 128 from a user, which may include an indication of a location of the ultrasound probe with respect to the lung region of the subject. In some examples, the user interface 122 may include a touch screen. The user interface may be configured to display various images 124, including the target image frame identified by the data processor 120, and at least one indicator 126. In some embodiments, the user interface 122 may display the target image frame simultaneously with a real-time, e.g., live, ultrasound image obtained via ultrasound echoes being actively received at the sensor array 112. The target image frame may thus appear as a still image and the real-time image may be dynamic. To reduce or avoid overlap with the real-time image, the user interface 122 may display the target image frame adjacent to the real-time image. Displaying the images side-by-side in this manner may facilitate user interpretation of the real-time images by minimizing any interference that may result from superimposing the target image frame on the real-time image.

In some examples, the user interface 122 may also be configured to display two or more sub-regions within the region being scanned. As described above, the data processor 120 is configured to identify one or more B-lines and a target image frame within each sub-region, each of which may be displayed by the user interface 122. For one or more of the sub-regions, the user interface 122 may display the number of B-lines detected by the data processor 120, their dimensions, start/end points, and/or intensity levels. Example user interfaces 122 may also display an indication of whether the number of B-lines exceeds a pre-determined threshold at any given sub-region. A B-line score may also be determined by the data processor 120 and displayed by the user interface 122 for each sub-region, such that a distribution of B-lines throughout the lung region may be displayed.

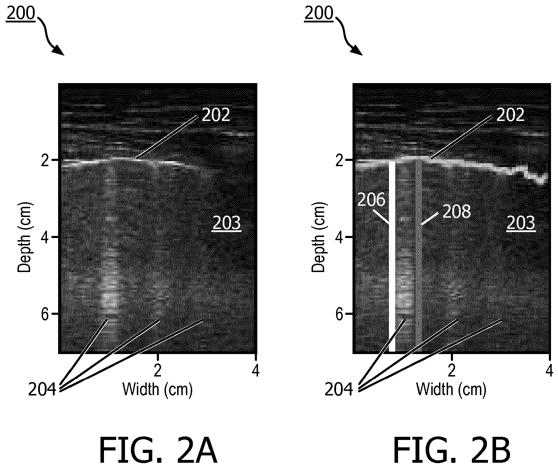

Figs. 2A-2D illustrate different aspects of the techniques employed by the system 100 to identify B-lines within an image frame. Fig. 2A is a B-mode image frame 200 that includes a pleural line 202, a region of interest 203 defined below the pleural line, and a plurality of candidate B-lines 204 within the region of interest. The image depth is displayed on the y-axis of the image, and the lateral width displayed on the x-axis. The image frame 200 may be generated by one or more processors, such as signal processor 118, in communication with an ultrasound data acquisition unit, e.g., data acquisition unit 110. As shown, the B-line candidates 204 may begin at the pleural line 202, at a depth of about 2 cm, and extend downward to a depth of approximately 7 cm.

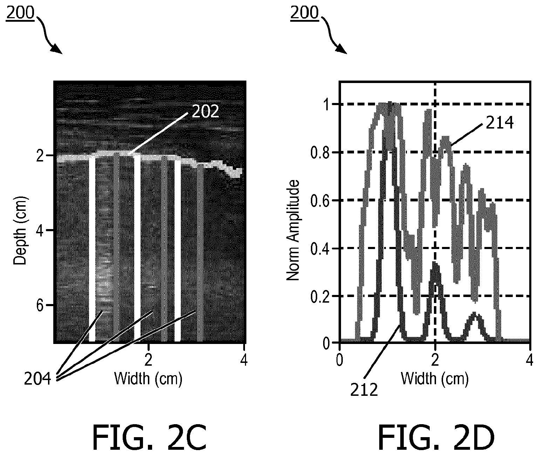

Fig. 2B illustrates the same image frame 200, including the pleural line 202 and the candidate B-lines 204. In Fig. 2B, the image frame 200 has been annotated such that the pleural line 202 and one of the candidate B-lines 204 has been identified. One or more processors, such as data processor 120, may be configured to identify these features on the image frame 200. In various examples, the data processor 120 may not actually mark the features on the image frame. Accordingly, the annotations shown in FIG. 2 may be provided for representation purposes only.

Fig. 2C also illustrates image frame 200, but with two additional B-line candidates 204 indicated. As shown, each of the candidate B-lines may appear as vertical, hyperechoic artifacts extending downward from the pleural line 202 into the region of interest 203.

Fig. 2D illustrates a graph 210 of a primary axial projection ("total AP") curve 212 and an averaged cross correlation ("CC") coefficient curve 214 for the B-line candidates 204 shown in FIGS. 2A-2C. In some examples, data processor 120 may be configured to generate the total AP curve 212 and averaged CC coefficient curve 214 to identify candidate B-lines 204 by determining a similarity metric across depth within each image frame. For example, if B-lines exist within the region of interest, they will most likely appear as peaks on the total AP curve, which may also be identified by the data processor 120. In some embodiments, each region of interest 203 within a single image frame may be divided into sub-regions by the data processor 120. The number of sub-regions may vary, ranging from about 4 to about 20 in different embodiments. For each sub-region within the region of interest 203, the data processor 120 may compute a sub-AP curve and compute a normalized CC coefficient between every sub-AP curve and the total AP curve 212. Higher CC values, e.g., any values or ranges above 0.7, 0.8, 0.9 or greater" may be used to narrow the pool of B-line candidates, as such values indicate greater similarity between each of the sub-band AP curves and the total AP curve, a result that may reflect the signature coherent properties (width, intensity, etc.) across image depth indicative of typical B-lines. Each of the three B-line candidates 204 identified in FIG. 2C correspond to a single peak on the total AP curve depicted in FIG. 2D.

Determining the normalized CC coefficient may reduce or eliminate discrepancies in the system's interpretation of B-lines detected at focal zones of different depth. For example, some sonographers may prefer focal zones located approximately at the pleural line, while others prefer focal zones at a depth of about 4 cm. Focal zones at different depths may impact the uniformity of the B-lines appearing in the plurality of image frames. Because B-line uniformity may be a key characteristic of B-line interpretation, it may be imperative that any variation stemming from different focal depths does not influence B-line identification. Normalizing the CC coefficient values may eliminate or at least minimize discrepancies. Measuring the similarity in B-line intensity at different sub-bands may also distinguish B-lines having intensity levels similar to background intensity levels in a given image frame. Generally, candidate B-lines and in some examples, legitimate B-lines by computing intensity similarity metrics according to the foregoing approaches may reduce computation loads such that, in some examples, portable ultrasound systems may be capable of identifying B-lines and/or target image frames.

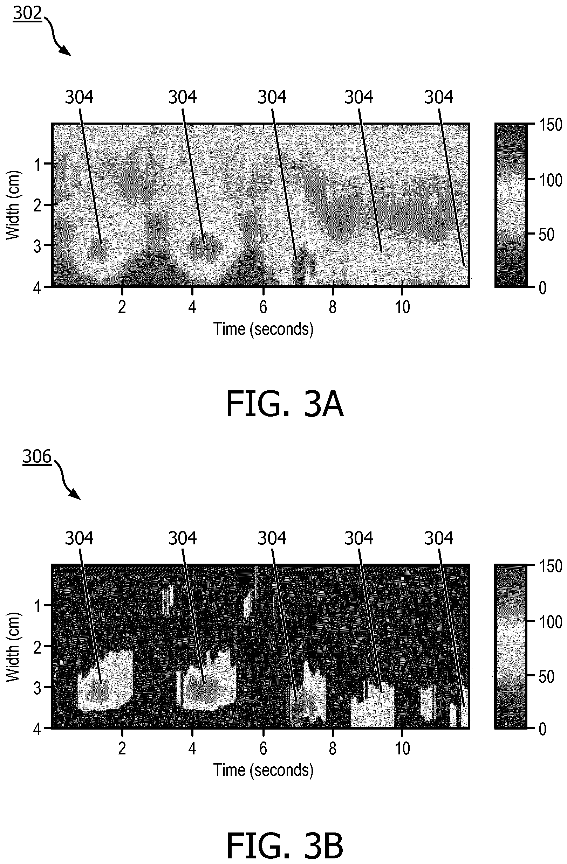

Figs. 3A and 3B illustrate graphical representations that may be produced, utilized, and/or displayed by the system 100 disclosed herein pursuant to selecting a target image frame from a plurality of image frames. In particular, FIGS. 3A and 3B illustrate the utilization of total intensity data gathered from a plurality of image frames to determine the time point, and depth, corresponding to the image frame of maximum overall intensity, which may correspond to the target image frame. Fig. 3A is an intensity map 302 showing axial intensity projections detected across multiple image frames as a function of lateral width (y-axis) and time (x-axis). As shown, the intensity map 302 includes multiple high-intensity regions 304. FIG. 3B is a line trace map 306 of the intensity map 302 shown in FIG. 3A, including the plurality of high-intensity regions 304. The line trace map 306 shows the location of axial intensity projections as a function of lateral width (y-axis) and time (x-axis). In line trace map 306, the image frames containing B-lines are indicated by removing variable shading from the axial intensity projections below a certain threshold. The threshold may thus be applied to eliminate areas of low intensity projection that likely do not represent image frames containing B-lines, or in some examples, image frames containing low numbers of B-lines or B-lines of low to moderate intensity.

In the examples shown, B-line intensity data is collected over a duration of about 12 seconds at a frame rate of 44 Hz, thus generating a total of 524 image frames, each of which is analyzed by data processor 120, for example, to determine axial intensity projection data for each frame. The intensity map shown in FIG. 3A thus represents a total axial projection curve map from all 524 image frames. The periodic pattern of B-line intensity portrayed in FIGS. 3A and 3B reflects the respiratory cycle. As shown, the strongest B-line intensity values are clustered around approximately 1.4 seconds. The target image frame selected by the data processor 120 may thus include the image frame generated at or near 1.4 seconds. One or more processors, such as data processor 120, may be configured to select the image frame generated at about 1.4 seconds, and communicate this image frame to a user interface, such as user interface 122, for display. In some examples, the image frame at 1.4 seconds may also be used to compute a B-line score. A target image frame may be selected over a fixed amount of time in some examples. In some embodiments, a target image frame may be selected over a fixed number of image frames and/or over a fixed number of respiration cycles. In addition or alternatively, the target image frame may be selected by summing the overall intensity levels of the all the pixels in a given image frame. The image frame with the highest summed intensity may represent the target image frame.

A user interface, such as user interface 122, may be configured to color-code areas of detected B-lines within a target image frame. The target image frame may be presented as a B-line trace map, such as the example shown in FIG. 3B, in some examples. Along with the location of each B-line, the user interface 122 may be configured to display the one or more B-line scores, a duration that each B-line remain across multiple image frames, and/or frame-to-frame differences in the B-line map.

Fig. 4 is a graphical representation of a B-line scoring system 400 according to embodiments of the present disclosure. The scoring system 400 is based on the proportion of B-line coverage within one or more intercostal spaces, and includes two variations: a linear scoring model 402 and a stepwise scoring model 404. Regardless of the specific scoring model employed, B-line(s) occupying the entirety of an intercostal space may be given the highest possible B-line score, e.g., 10, while the absence of B-lines within an intercostal score may correspond with the lowest possible B-line score, e.g., 0. Scores generated by the continuous model 402 may capture smaller variations in B-line coverage compared to the stepwise model 404. For instance, as shown in FIG. 4, the B-line score calculated according to the stepwise model remains constant between 20% and 30% B-line coverage, while the B-line score calculated over the same coverage range according to the continuous model 402 increases continuously as the proportion of B-line coverage increases.

Intercostal spaces may be determined according to various methods. For example, during a longitudinal scan, an intercostal space may be defined as the width between two adjacent ribs based on the actual length of the pleural line spanning the intercostal space, or the between the two boundaries of the shadows created by the ribs. For transverse scans, an intercostal space may be defined as the actual length of the pleural line shown on a B-mode image.

One or more B-line scores are calculated based on the number of discrete B-lines present within one or more intercostal spaces. In such examples, a discrete B-line width may be defined. The standard width of a single B-line may be defined as, for instance, less than 50% of the width of the intercostal space, which may be approximately 2 cm. The number of discrete B-lines having a width of less than 50% may be equal to, or directly related to, the overall B-lines score. For example, 4 B-lines detected within a single intercostal space may correspond to a B-line score of 4 (on a scale of 1 to 10). B-lines of greater width may indicate fused and/or confluent B-lines, which may be assigned a score based on the fused/confluent width. For instance, if the width is greater than 50%, but less than 75% of the intercostal space, the fused and/or confluent B-line may be assigned a score of 6. If the width is greater than 75%, but less than 100%, the fused/confluent B-line may be given a score of 7. If the width is 100% of the intercostal space, then the fused/confluent B-line may be given a score of 8, and so on.

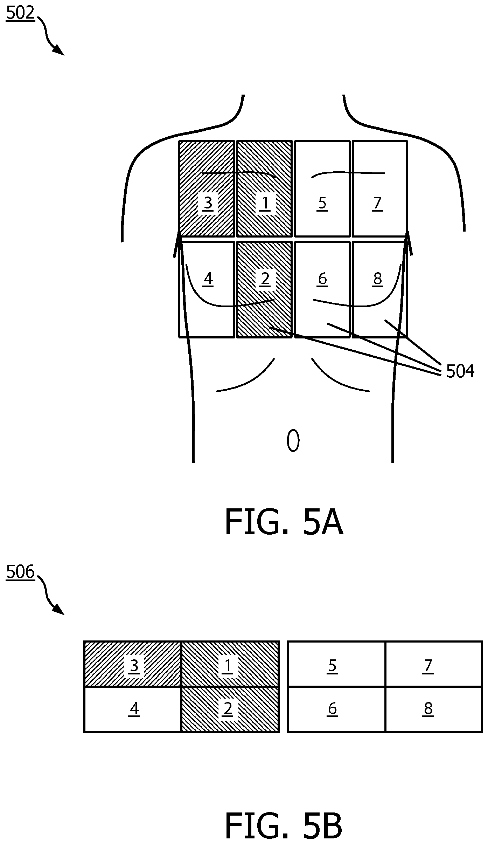

Fig. 5A is a diagram of multiple zones or sub-regions within a region of interest of a subject. The diagram 502 shown in Fig. 5A may be generated and displayed on a user interface, for example user interface 122. The diagram 502 includes a schematic of the chest region of a subject, which is divided into 8 separate zones or sub-regions 504. One or more sub-regions 504 may be selectable by a user, e.g., via a touch interface. Upon selecting a sub-region 504, the user interface 122 may display various types of statistical information regarding the B-lines detected within that particular sub-region, e.g., the number of B-lines detected and/or one or more B-line scores. The particular embodiment shown in Fig. 5A includes 8 sub-regions 504. More or fewer than 8 sub-regions may be included in some examples. Three of the sub-regions 504 (sub-regions #1, #2 and #3) shown in Fig. 5A may be shaded a different color than the rest of the sub-regions. This may indicate, in some embodiments, that these three zones have already been selected for examination by a user. FIG. 5B is a table reporting a qualitative B-line score for each sub-region shown in Fig. 5A. The table 506 may report B-line scores by assigning different colors to different scores. For example, sub-region #3 may be shaded red to indicate a high B-line score, e.g., a score equal to or greater than 5. By contrast, sub-regions #1 and #2 may be shaded green or blue to indicate a low B-line score, e.g., a score equal to or greater than 0 but less than 3. Color coding may be determined by a data processor, e.g., data processor 120.

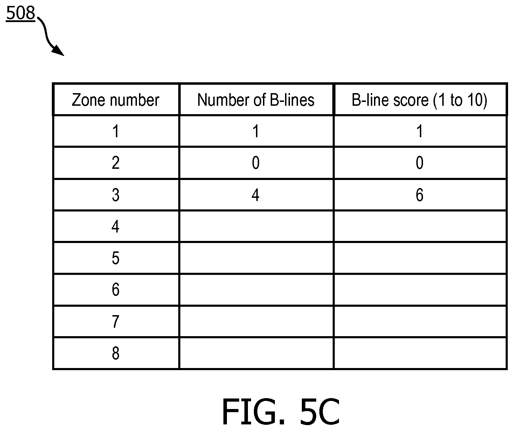

Fig. 5C is a table reporting a quantitative B-line score for each region of interest shown in Fig. 5A. The example table 508 shown in FIG. 5C includes 3 columns: a first column indicating the sub-region numbers shown in the diagram 502 illustrated in FIG. 5A, a second column indicating the number of B-lines detected at the particular sub-region numbers, and a third column showing the calculated B-line score for each sub-region on a scale of 1 to 10. Consistent with the table 506 shown in Fig. 5B, table 508 confirms that zone #3 includes the highest B-line score (6) of the sub-regions evaluated. The quantitative data included in table 508 may inform estimations of extravascular lung water and/or may facilitate precise monitoring of a condition, e.g., pulmonary edema, over time.

One or more of Figs. 5A-5C may be displayed on a given user interface simultaneously. In some examples, a user may toggle between images on the user interface, for example switching between a table view, a diagram view, a quantitative view, a qualitative view, and/or a live image feed. In some embodiments, a user interface may be configured to receive manual input directly into one or more tables, such as the table 508 shown in FIG. 5C. After populating a table containing B-line information, the table may be archived for later viewing.

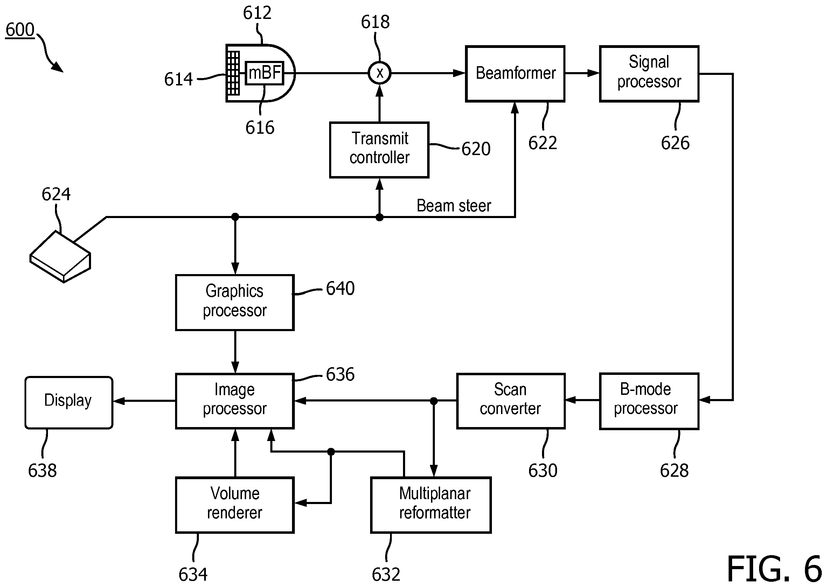

Fig. 6 illustrates an ultrasound imaging system 600 constructed in accordance with the principles of the present invention. One or more components shown in FIG. 6 may be included within a system configured to identify B-lines within a region of a subject, select a target image frame containing an image of the B-lines, and/or display the target image frame, along with B-line scores and/or statistics, to a user. For example, any of the above-described functions of the signal processor 118 and the data processor 120 may be programmed, e.g., via computer executable instructions, into an existing processor of the system 600. In some examples, the functions of the data processor 120 may be implemented and/or controlled by one or more of the processing components shown in FIG. 6, including for example, the B-mode processor 628, scan converter 630, multiplanar reformatter 632, volume renderer 634 and/or image processor 636.

In the ultrasonic imaging system of Fig. 6, an ultrasound probe 612 includes a transducer array 614 for transmitting ultrasonic waves into a region containing the lungs and receiving echo information responsive to the transmitted waves. The transducer array 614 may be a matrix array that includes a plurality of transducer elements configured to be individually activated. In other embodiments, the transducer array 614 may be a one-dimensional linear array. The transducer array 614 is coupled to a microbeamformer 616 in the probe 612 which may control the transmission and reception of signals by the transducer elements in the array. In the example shown, the microbeamformer 616 is coupled by the probe cable to a transmit/receive (T/R) switch 618, which switches between transmission and reception and protects the main beamformer 622 from high energy transmit signals. In some embodiments, the T/R switch 618 and other elements in the system can be included in the transducer probe rather than in a separate ultrasound system base. The transmission of ultrasonic beams from the transducer array 614 under control of the microbeamformer 616 is directed by the transmit controller 620 coupled to the T/R switch 618 and the beamformer 622, which receives input, e.g., from the user's operation of the user interface or control panel 624. One of the functions controlled by the transmit controller 620 is the direction in which beams are steered. Beams may be steered straight ahead from (orthogonal to) the transducer array, or at different angles for a wider field of view. The partially beamformed signals produced by the microbeamformer 616 are coupled to a main beamformer 622 where partially beamformed signals from individual patches of transducer elements are combined into a fully beamformed signal.

The beamformed signals are coupled to a signal processor 626. Signal processor 626 may process the received echo signals in various ways, such as bandpass filtering, decimation, I and Q component separation, and harmonic signal separation. Data generated by the different processing techniques employed by the signal processor 626 may be used by a data processor to identify pleural lines, B-lines, or internal structures, e.g., ribs, and parameters thereof. The signal processor 626 may also perform additional signal enhancement such as speckle reduction, signal compounding, and noise elimination. The processed signals may be coupled to a B-mode processor 628, which can employ amplitude detection for the imaging of structures in the body, including the ribs, the heart, and/or the pleural interface, for example. The signals produced by the B-mode processor are coupled to a scan converter 630 and a multiplanar reformatter 632. The scan converter 630 arranges the echo signals in the spatial relationship from which they were received in a desired image format. For instance, the scan converter 630 may arrange the echo signals into a two dimensional (2D) sector-shaped format. The multiplanar reformatter 632 can convert echoes which are received from points in a common plane in a volumetric region of the body into an ultrasonic image of that plane, as described in U.S. Pat. No. 6,443,896 (Detmer). A volume renderer 634 converts the echo signals of a 3D data set into a projected 3D image as viewed from a given reference point, e.g., as described in U.S. Pat. No. 6,530,885 (Entrekin et al.). The 2D or 3D images are coupled from the scan converter 630, multiplanar reformatter 632, and volume renderer 634 to an image processor 636 for further enhancement, buffering and temporary storage for display on an image display 638. The graphics processor 636 can generate graphic overlays for display with the ultrasound images. These graphic overlays can contain, e.g., standard identifying information such as patient name, date and time of the image, imaging parameters, and the like, and also various B-line statistics and/or B-line scores. Graphic overlays may also include one or more signals indicating the target image frame has been obtained and/or the system 600 is in the process of identifying the target image frame. The graphics processor may receive input from the user interface 624, such as a typed patient name. The user interface 624 may also receive input prompting adjustments in the settings and/or parameters used by the system 600. The user interface can also be coupled to the multiplanar reformatter 632 for selection and control of a display of multiple multiplanar reformatted (MPR) images.

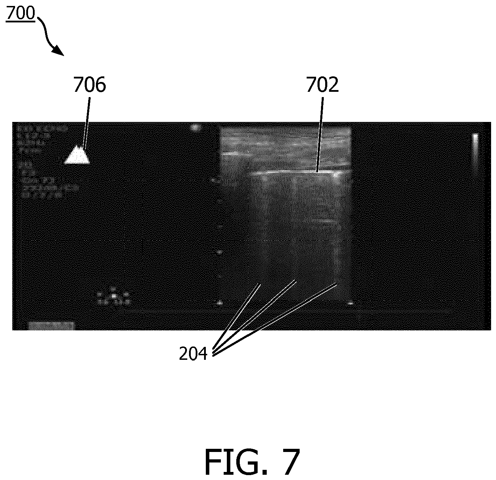

Fig. 7 is a lung ultrasound image 700 of a lung region taken at a target image frame selected according to the embodiments described herein. The image 700 shown in FIG. 7 may be obtained with a linear array transducer in a tissue-harmonic imaging mode. The image 700 includes a pleural line 702 and three B-lines 704, each B-line beginning at the pleural line 702 and extending downward. FIG. 7 also depicts an example of an indicator 706 displayed on the screen to provide one or more indications regarding the detected B-lines and/or the implications of such B-lines. In various examples, the indicator 706 may indicate to a user that the target image frame is being displayed. The indicator 706 may indicate to a user that the number of B-lines depicted in the image 700 satisfies one or more threshold numbers of B-lines. For example, the number of B-lines may indicate the existence and/or volume of extravascular lung water. The indicator 706 may appear and/or undergo a change in appearance if 3 or more B-lines are identified in a given image, for example, to alert a user that extravascular lung water may be present within the lungs. In some embodiments, the indicator 706 may indicate to a user that the lung region depicted in the image is either normal or abnormal. The indicator 706 may comprise an absolute, binary signal that either appears on the image or does not appear, or the indicator 706 may provide a gradual signal that changes based on the intensity of the B-lines included in an image frame. For example, as the number of B-lines increase above a certain threshold, the severity of extravascular lung water may intensify. The indicator 706 may reflect this gradual change in intensity by changing in brightness or color, for example. In some embodiments, the indicator 706 may not include a displayed graphic at all, instead including an audio cue and/or tactile stimulation, for example. In some examples, additional information may be displayed on the image 700. For example, an indication of whether the number of B-lines shown in the image 700 satisfies a given threshold may be included.

The information conveyed in a target image frame, such as that depicted in Fig. 7, may be utilized pursuant to a variety of applications. For example, PTX may be diagnosed with up to 100% specificity based on the presence or absence of B-lines at a given location. Specifically, the presence of B-lines may be used to rule out the possibility of diagnosing a patient with PTX. Various fluid therapies may also be guided with information regarding extravascular lung water volumes and locations determined based on the number of B-lines, width of B-lines, and/or average distances between any pair of B-lines.

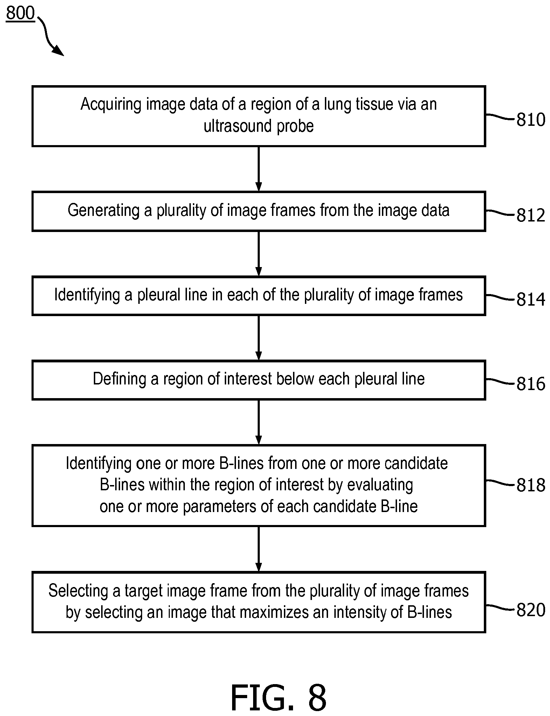

Fig. 8 is a block diagram of an ultrasound imaging method in accordance with the principles of the present disclosure. The example method 800 of Fig. 8 shows the steps that are utilized, in any sequence, by the systems and/or apparatuses described herein for identifying B-lines and selecting a target image frame containing the B-lines for display. The method 800 is performed by an ultrasound imaging system, such as system 800, or other systems including, for example, a mobile system such as LUMIFY by Philips. Additional example systems may include SPARQ and/or EPIQ, also produced by Philips.

The method 800 begins at block 810 by "acquiring image data of a region of a lung tissue via an ultrasound probe." Image data may be gathered via an ultrasound data acquisition unit, which may contain various configurations of sensor arrays, including those described above with respect to FIG. 1. The region may span the entire chest region or at least one or more portions thereof. One or more locations within the region may be targeted by a user operating the ultrasound data acquisition unit if such locations have been identified previously as harboring one or more abnormalities, example a pulmonary edema and/or extravascular lung water.

At block 812, the method involves "generating a plurality of image frames from the image data." The image frames may be generated by processing ultrasound echoes received at the data acquisition unit. A collection of image frames may be collected in series at various discrete locations throughout the region of the lung tissue being imaged. One or more of the image frames may be captured and stored by one or more devices configured to perform the method 800. One or more of the image frames may include at least one pleural line and one or more B-lines. In some examples, none of the image frames may include a B-line.

At block 814, the method involves "identifying a pleural line in each of the plurality of image frames." Various techniques may be employed, for example by a data processor, to identify pleural lines. Such techniques may apply intensity thresholding to identify the presence and/or boundaries of one or more pleural lines.

At block 816, the method involves "defining a region of interest below each pleural line." The region of interest includes the area beneath, i.e., at greater depths, the pleural line. If one or more B-lines are present in a given image frame, the B-lines will appear beneath the pleural line. Regions of interest may be of uniform dimensions in some examples. In some embodiments, the size of the regions of interest may vary across multiple image frames and/or across various implementations of the method 800.

At block 818, the method involves "identifying one or more B-lines from one or more candidate B-lines within the region of interest by evaluating one or more parameters of each candidate B-line." In some examples, candidates may be identified by determining an intensity similarity metric across depth within each image frame. In some embodiments, candidate B-lines may be selected by measuring axial intensity projection data across one or more sub-regions within the region of interest defined within each individual image frame. Cross correlation coefficients may be normalized across multiple sub-bands to evaluate the likelihood that peaks in the axial projection intensity data correspond to B-lines. In some embodiments, B-lines may be identified by measuring one or more parameters indicative of most B-lines. In various examples, the parameters may include an intensity uniformity level, a length, a starting location, an ending location, and/or a level of the motion detected. For instance, B-lines typically begin at the pleural line. In addition, B-lines commonly appear uniform in intensity and dimension along the length of each B-line. B-lines may also extend from the pleural line to the bottom of the region of interest.

At block 820, the method involves "selecting a target image frame from the plurality of image frames by selecting an image frame that maximizes an intensity of B-lines." In some examples, the target image frame may be the frame having the highest calculated B-line score within a series of image frames. The B-line score being based on the proportion of an intercostal space covered by one or more B-lines. The target image frame corresponds to the image frame having the highest overall intensity compared to a plurality of image frames.

The method 800 may further involve, for example, displaying the target image frame simultaneously with a real-time image of the lung tissue and/or comparing two or more image frames to detect motion of one or more candidate B-lines. In some embodiments, the method 800 involves determining a proportion of each intercostal space that is covered by one or more B-lines, and generating a B-line score based on the proportion for each intercostal space and may involve determining an intercostal space between each pair of ribs within the region of interest. Such methods may also involve generating and displaying a pictorial representation of multiple B-line scores, each B-line score corresponding to a sub-region within the region of the lung tissue.

Of course, it is to be appreciated that any one of the examples, embodiments or processes described herein may be combined with one or more other examples, embodiments and/or processes or be separated and/or performed amongst separate devices or device portions in accordance with the present systems, devices and methods. The above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended scope of the present system as set forth in the claims that follow. Accordingly, the specification and drawings are to be regarded in an illustrative manner and are not intended to limit the scope of the appended claims.

## Claims

1. An ultrasound imaging system (100, 600) comprising:
an ultrasound probe (612) configured to receive ultrasound echoes (115) from a subject to image a lung region of the subject;
at least two processors (118, 120), in communication with the ultrasound probe, that:
generate (812) a plurality of image frames (119) from the ultrasound echoes;
identify (814) a pleural line (702) in each of the plurality of image frames;
define (816) a region of interest (203) below each pleural line;
identify (818) one or more B-lines (704) from one or more candidate B-lines within the region of interest by evaluating one or more parameters of each candidate B-line; and
select (820) a target image frame from the plurality of image frames by identifying an image frame providing a maximal intensity of identified B-lines, wherein the intensity of B-lines comprises a B-line score, the B-line score based at least in part on a level of B-line coverage within at least one intercostal space present within the region of interest,
**characterized in that** the one or more parameters comprise a level of motion detected across multiple image frames.

2. The ultrasound imaging system of claim 1, wherein the one or more parameters further comprise at least one of a level of intensity uniformity, a length, a starting location, an ending location.

3. The ultrasound imaging system of claim 1 or 2, further comprising a user interface (122) in communication with at least one of the processors, the user interface configured to display the target image frame simultaneously with a real-time image responsive to the ultrasound echoes received at the ultrasound probe.

4. The ultrasound imaging system of claim 3, wherein the user interface is configured to display two or more sub-regions (504) selectable by a user, each sub-region corresponding to a portion of the lung region of the subject.

5. The ultrasound imaging system of claim 4, wherein the processors are further configured to identify one or more B-lines and a target frame within each sub-region.

6. The ultrasound imaging system of claim 5, wherein for each sub-region, the user interface is configured to display one or more of a number of B-lines, an indication of whether the number of B-lines exceeds a pre-determined threshold, or a starting and ending location of each B-line.

7. The ultrasound imaging system of claims 5 or 6, wherein the processors are further configured to determine a B-line score for each sub-region, the B-line score based at least in part on a level of B-line coverage within at least one intercostal space present within each sub-region.

8. The ultrasound imaging system of claim 7, wherein the user interface is configured to provide an indication of the B-line score and an indication of whether the B-line score is normal or abnormal for each sub-region such that a distribution of B-lines throughout the lung region is displayed.

9. The ultrasound imaging system of any one of claims 1 to 8, wherein the intensity of B-lines comprises at least one of a number of B-lines or a width of one or more B-lines.

10. The ultrasound imaging system of any one of claims 1 to 9, wherein the processors are configured to identify one or more candidate B-lines by generating an axial projection curve within the region of interest, wherein the axial projection provides a normalized value of the candidate B-line intensity at a corresponding lateral width.

11. The ultrasound imaging system of claim 10, wherein the processors are further configured to generate two or more sub-axial projection curves within two or more sub-locations within the region of interest and determine a normalized cross correlation coefficient between each of the sub-axial projection curves and the axial projection curve.

12. A method comprising:
acquiring (810) image data of a region of a lung tissue via an ultrasound probe;
generating (812) a plurality of image frames from the image data;
identifying (814) a pleural line in each of the plurality of image frames;
defining (816) a region of interest below each pleural line;
identifying (818) one or more B-lines from one or more candidate B-lines within the region of interest by evaluating one or more parameters of each candidate B-line; and
selecting (820) a target image frame from the plurality of image frames by selecting an image frame providing a maximal intensity of B-lines, wherein the intensity of B-lines comprises a B-line score, the B-line score based at least in part on a level of B-line coverage within at least one intercostal space present within the region of interest,
the method **characterized by** comparing two or more image frames to detect motion of one or more candidate B-lines.

13. The method of claim 12, further comprising displaying the target image frame simultaneously with a real-time image of the lung tissue.

14. The method of claim 12, wherein the one or more parameters comprise at least one of an intensity uniformity level, a length, a starting location, an ending location, or a level of the motion detected.

15. The method of any one of claims 12 to 14, further comprising:
identifying an intercostal space between at least one pair of ribs within the region of interest;
determining a proportion of the intercostal space covered by one or more B-lines; and
generating a B-line score based on the proportion.

## Patentansprüche

1. Ultraschall-Bildgebungssystem (100, 600), umfassend:
eine Ultraschallsonde (612), die so konfiguriert ist, dass sie Ultraschallechos (115) von einem Subjekt empfängt, um eine Lungenregion des Subjekts abzubilden;
mindestens zwei Prozessoren (118, 120), die mit der Ultraschallsonde kommunizieren, so dass:
Erzeugen (812) einer Vielzahl von Bildrahmen (119) aus den Ultraschallechos;
Identifizieren (814) einer Pleuralinie (702) in jedem der Vielzahl von Bildrahmen;
Definieren (816) eines interessierenden Bereichs (203) unterhalb jeder Pleuralinie;
Identifizieren (818) einer oder mehrerer B-Linien (704) von einem oder mehreren Kandidaten-B-Linien innerhalb des interessierenden Bereichs durch Auswerten eines oder mehrerer Parameter jeder Kandidaten-B-Linie; und
Auswählen (820) eines Zielbildrahmens aus der Vielzahl von Bildrahmen durch Identifizieren eines Bildrahmens, der eine maximale Intensität der identifizierten B-Linien bereitstellt, wobei die Intensität der B-Linien eine B-Linien-Bewertung umfasst, wobei die B-Linien-Bewertung zumindest teilweise auf einem Grad der B-Linien-Abdeckung in mindestens einem Interkostalraum innerhalb des interessierenden Bereichs basiert,
**dadurch gekennzeichnet, dass** der eine oder die mehreren Parameter einen Bewegungsgrad umfassen, das über mehrere Bildrahmen hinweg erfasst wird.

2. Ultraschall-Bildgebungssystem nach Anspruch 1, wobei der eine oder die mehreren Parameter außerdem mindestens einen von einem Grad der Intensitätsgleichmäßigkeit, einer Länge, einem Startort und einem Endort umfassen.

3. Ultraschall-Bildgebungssystem nach Anspruch 1 oder 2, ferner umfassend eine Benutzerschnittstelle (122), die mit mindestens einem der Prozessoren kommuniziert, wobei die Benutzerschnittstelle so konfiguriert ist, dass sie als Reaktion auf die an der Ultraschallsonde empfangenen Ultraschallechos den Zielbildrahmen gleichzeitig mit einem Echtzeitbild anzeigt.

4. Ultraschall-Bildgebungssystem nach Anspruch 3, wobei die Benutzeroberfläche so konfiguriert ist, dass sie zwei oder mehr von einem Benutzer auswählbare Unterregionen (504) anzeigt, wobei jede Unterregion einem Teil der Lungenregion des Subjekts entspricht.

5. Ultraschall-Bildgebungssystem nach Anspruch 4, wobei die Prozessoren außerdem konfiguriert sind, um eine oder mehrere B-Linien und einen Zielrahmen innerhalb jedes Unterbereichs zu identifizieren.

6. Ultraschall-Bildgebungssystem nach Anspruch 5, wobei die Benutzerschnittstelle für jeden Unterbereich so konfiguriert ist, dass sie eine oder mehrere einer Anzahl von B-Linien, einen Hinweis darauf, ob die Anzahl der B-Linien einen vorgegebenen Schwellenwert überschreitet, oder einen Start- und Endort jeder B-Linie anzeigt.

7. Ultraschall-Bildgebungssystem nach Anspruch 5 oder 6, wobei die Prozessoren außerdem konfiguriert sind, um für jede Unterregion eine B-Linien-Bewertung zu ermitteln, wobei die B-Linien-Bewertung zumindest teilweise auf einem Grad der B-Linien-Abdeckung innerhalb mindestens eines Interkostalraums basiert, der in jeder Unterregion vorhanden ist.

8. Ultraschallbildgebungssystem nach Anspruch 7, wobei die Benutzerschnittstelle so konfiguriert ist, dass sie für jede Unterregion eine Anzeige der B-Linien-Bewertung und eine Anzeige darauf bereitstellt, ob die B-Linien-Bewertung normal oder abnormal ist, sodass eine Verteilung der B-Linien im gesamten Lungenbereich angezeigt wird.

9. Ultraschallbildgebungssystem nach einem der Ansprüche 1 bis 8, wobei die Intensität der B-Linien mindestens eine von mehreren B-Linien oder eine Breite von einer oder mehreren B-Linien umfasst.

10. Ultraschallbildgebungssystem nach einem der Ansprüche 1 bis 9, wobei die Prozessoren so konfiguriert sind, dass sie eine oder mehrere Kandidaten-B-Linien identifizieren, indem sie eine axiale Projektionskurve innerhalb des interessierenden Bereichs erzeugen, wobei die axiale Projektion einen normalisierten Wert der Intensität der Kandidaten-B-Linie bei einer entsprechenden lateralen Breite liefert.

11. Ultraschall-Bildgebungssystem nach Anspruch 10, wobei die Prozessoren weiterhin so konfiguriert sind, dass sie zwei oder mehr subaxiale Projektionskurven innerhalb von zwei oder mehr Suborten innerhalb des interessierenden Bereichs erzeugen, und einen normalisierten Kreuzkorrelationskoeffizienten zwischen jeder der subaxialen Projektionskurven und der axialen Projektionskurve bestimmt.

12. Verfahren, umfassend:
Erfassen (810) von Bilddaten eines Bereichs eines Lungengewebes über eine Ultraschallsonde;
Erzeugen (812) einer Vielzahl von Bildrahmen aus den Bilddaten;
Identifizieren (814) einer Pleuralinie in jedem der Vielzahl von Bildrahmen;
Definieren (816) eines interessierenden Bereichs unterhalb jeder Pleuralinie;
Identifizieren (818) einer oder mehrerer B-Linien aus einem oder mehreren Kandidaten-B-Linien innerhalb des interessierenden Bereichs durch Auswerten eines oder mehrerer Parameter jeder Kandidaten-B-Linie; und
Auswählen (820) eines Zielbildrahmens aus der Vielzahl von Bildrahmen durch Auswählen eines Bildrahmens, der eine maximale Intensität von B-Linien bereitstellt, wobei die Intensität von B-Linien eine B-Linien-Bewertung umfasst, wobei die B-Linien-Bewertung zumindest teilweise auf einem Grad der B-Linien-Abdeckung in mindestens einem Interkostalraum innerhalb der interessierenden Region basiert,
wobei das Verfahren **dadurch gekennzeichnet ist, dass** zwei oder mehr Bildrahmen verglichen werden, um die Bewegung einer oder mehrerer Kandidaten-B-Linien zu erfassen.

13. Verfahren nach Anspruch 12, das außerdem das gleichzeitige Anzeigen des Zielbildrahmens mit einem Echtzeitbild des Lungengewebes umfasst.

14. Verfahren nach Anspruch 12, wobei der eine oder die mehreren Parameter mindestens einen von einem Intensitätsgleichmäßigkeitsgrad, einer Länge, einem Startort, ein Endort oder ein Grad der erfassten Bewegung.

15. Verfahren nach einem der Ansprüche 12 bis 14, weiterhin umfassend:
Identifizieren eines Interkostalraums zwischen mindestens einem Rippenpaar innerhalb des interessierenden Bereichs;
Bestimmen eines Anteils des Interkostalraums, der von einer oder mehreren B-Linien abgedeckt wird; und
Erzeugen einer B-Line-Bewertung basierend auf dem Anteil.

## Revendications

1. Système d'imagerie par ultrasons (100, 600) comprenant :
une sonde à ultrasons (612) configurée pour recevoir des échos ultrasonores (115) provenant d'un sujet afin d'imager une région pulmonaire du sujet;
au moins deux processeurs (118, 120), en communication avec la sonde à ultrasons, qui:
génèrent (812) une pluralité de trames d'images (119) à partir des échos ultrasonores;
identifient (814) une ligne pleurale (702) dans chacune de la pluralité de trames d'image;
définissent (816) une région d'intérêt (203) au-dessous de chaque ligne pleurale;
identifient (818) une ou plusieurs lignes B (704) parmi une ou plusieurs lignes B candidates dans la région d'intérêt en évaluant un ou plusieurs paramètres de chaque ligne B candidate; et
sélectionnent (820) une trame d'image cible parmi la pluralité de trames d'image en identifiant une trame d'image fournissant une intensité maximale de lignes B identifiées, où l'intensité des lignes B comprend un score des lignes B, le score des lignes B étant basé au moins en partie sur un niveau de couverture de lignes B dans au moins un espace intercostal présent dans la région d'intérêt,
**caractérisé en ce que** l'un ou plusieurs paramètres comprennent un niveau de mouvement détecté sur des multiples trames d'image.

2. Système d'imagerie par ultrasons selon la revendication 1, dans lequel l'un ou plusieurs paramètres comprennent en outre au moins un niveau d'uniformité d'intensité, une longueur, un emplacement de début, et un emplacement de fin.

3. Système d'imagerie par ultrasons selon la revendication 1 ou 2, comprenant en outre une interface utilisateur (122) en communication avec au moins l'un des processeurs, l'interface utilisateur étant configurée pour afficher la trame d'image cible simultanément avec une image en temps réel en réponse aux échos ultrasonores reçus au niveau de la sonde à ultrasons.

4. Système d'imagerie par ultrasons selon la revendication 3, dans lequel l'interface utilisateur est configurée pour afficher deux ou plusieurs sous-régions (504) sélectionnables par un utilisateur, chaque sous-région correspondant à une partie de la région pulmonaire du sujet.

5. Système d'imagerie par ultrasons selon la revendication 4, dans lequel les processeurs sont en outre configurés pour identifier une ou plusieurs lignes B et une trame cible dans chaque sous-région.

6. Système d'imagerie par ultrasons selon la revendication 5, dans lequel pour chaque sous-région, l'interface utilisateur est configurée pour afficher une ou plusieurs parmi un certain nombre de lignes B, une indication montrant si le nombre de lignes B dépasse un seuil prédéterminé, ou un emplacement de début et de fin de chaque ligne B.

7. Système d'imagerie par ultrasons selon les revendications 5 ou 6, dans lequel les processeurs sont en outre configurés pour déterminer un score des lignes B pour chaque sous-région, le score des lignes B étant basé au moins en partie sur un niveau de couverture des lignes B dans au moins un espace intercostal présent dans chaque sous-région.

8. Système d'imagerie par ultrasons selon la revendication 7, dans lequel l'interface utilisateur est configurée pour fournir une indication du score des lignes B et une indication montrant si le score des lignes B est normal ou anormal pour chaque sous-région de telle sorte qu'une distribution des lignes B dans toute la région pulmonaire soit affichée.

9. Système d'imagerie par ultrasons selon l'une quelconque des revendications 1 à 8, dans lequel l'intensité des lignes B comprend au moins une parmi un certain nombre de lignes B ou une largeur d'une ou plusieurs lignes B.

10. Système d'imagerie par ultrasons selon l'une quelconque des revendications 1 à 9, dans lequel les processeurs sont configurés pour identifier une ou plusieurs lignes B candidates en générant une courbe de projection axiale dans la région d'intérêt, où la projection axiale fournit une valeur normalisée de l'intensité de ligne B candidate à une largeur latérale correspondante.

11. Système d'imagerie par ultrasons selon la revendication 10, dans lequel les processeurs sont en outre configurés pour générer deux ou plusieurs courbes de projection sous-axiale dans deux ou plusieurs sous-emplacements dans la région d'intérêt et déterminer un coefficient de corrélation croisée normalisé entre chacune des courbes de projection sous-axiale et la courbe de projection axiale.

12. Procédé consistant à:
acquérir (810) des données d'image d'une région d'un tissu pulmonaire via une sonde à ultrasons;
générer (812) une pluralité de trames d'image à partir des données d'image;
identifier (814) une ligne pleurale dans chacune de la pluralité de trames d'image;
définir (816) une région d'intérêt au-dessous de chaque ligne pleurale;
identifier (818) une ou plusieurs lignes B parmi une ou plusieurs lignes B candidates dans la région d'intérêt en évaluant un ou plusieurs paramètres de chaque ligne B candidate; et
sélectionner (820) une trame d'image cible parmi la pluralité de trames d'image en sélectionnant une trame d'image fournissant une intensité maximale de lignes B, où l'intensité des lignes B comprend un score des lignes B, le score des lignes B étant basé au moins en partie sur un niveau de couverture de lignes B dans au moins un espace intercostal présent dans la région d'intérêt,
le procédé étant **caractérisé par** la comparaison de deux ou plusieurs trames d'image pour détecter le mouvement d'une ou plusieurs lignes B candidates.

13. Procédé selon la revendication 12, comprenant en outre l'affichage de la trame d'image cible simultanément avec une image en temps réel du tissu pulmonaire.

14. Procédé selon la revendication 12, dans lequel l'un ou plusieurs paramètres comprennent au moins un niveau d'uniformité d'intensité, une longueur, un emplacement de début, un emplacement de fin ou un niveau de mouvement détecté.

15. Procédé selon l'une quelconque des revendications 12 à 14, consistant en outre à:
identifier un espace intercostal entre au moins une paire de côtes dans la région d'intérêt;
déterminer une proportion de l'espace intercostal couvert par une ou plusieurs lignes B; et
générer un score des lignes B basé sur la proportion.
